# EUROPEAN PATENT SPECIFICATION

(11) **EP 0 528 827 B1**
(45) Date of publication and mention of the grant of the patent: **04.09.1996**
(21) Application number: 91908131.5
(22) Date of filing: 26.03.1991
(51) Int. Cl.: C12N 15/12, C12N 15/33, C12N 15/73

(54) **PROTEIN PARTNER SCREENING ASSAYS AND USES THEREOF**
SCREENING NACH PROTEINPARTNERN UND DEREN VERWENDUNG
TECHNIQUES DE TRIAGE DE PARTENAIRES DE PROTEINES ET LEURS UTILISATIONS

(30) Priority: 19.04.1990 US 510254
(43) Date of publication of application: 03.03.1993
(73) Proprietor: THE GENERAL HOSPITAL CORPORATION, Boston, MA 02114 (US)
(72) Inventor: KINGSTON, Robert, E., Arlington, MA 02174 (US)
(74) Representative: Aulmich, Gerhard, Dr.
(86) International application number: US9102076
(87) International publication number: WO9116429

(56) References cited:
- WO-A-89/07654
- WO-A-91/16456
- WO-A-92/05286
- CELL, vol. 52, 29 January 1988, Cell Press, Cambridge, NA.; pages 179 - 184, K. LECH et al. 'DNA-bound Fos proteins activate transcription in yeast'
- PROC. NATL. ACAD. SCI., vol. 86, May 1989, NATL. ACAD. SCI., Washington, DC, US; pp. 3689-3693, S.J. Elledge et al.
- Proceedings of the National Academy of Sciences, Vol. 85, issued August 1988, HOLLIS et al., "A repressor heterodimer binds to a chimeric operator", pp. 5834-5839
- The EMBO Journal, Vol. 1, No. 5, issued May 1982, GICQUEL-SANZEY et al., "Homologies between different procaryotic DNA-binding regulatory proteins and between their sites of action", pages 591 to 595
- Nature, Vol. 316, issued 15 August 1985, WHARTON et al., "Changing the binding specificity of a repressor by redesigning an alpha-helix", pages 601 to 605

## Description

### Field of the Invention

This invention is in the field of molecular biology and is directed to a method of identifying a peptide capable of associating with another peptide in a heterodimeric complex. The invention is also directed to a method of identifying inhibitors of such heterodimeric complex formation.

### BACKGROUND OF THE INVENTION

### I. Oncogenes

The induction of many types of cancer is thought to be ultimately caused by activation of cellular oncogenes (Bishop, J.M., *Science 235*:305-310 (1987); Barbacid, M., *Ann. Rev. Biochem. 56*:779-827 (1987); Cole, M.D., *Ann. Rev. Genet. 20*:361-384 (1986); and Weinberg, R.A., *Science 230*:770-776 (1985)). Such oncogenes express oncoproteins that reside in the cell, often localized to a specific site such as the nucleus, cytoplasm or the cell membrane.

For example, the cellular c-myc oncogene encodes the c-myc proteins. Expression of large amounts of c-myc in a variety of cell types allows cells to grow indefinitely in culture (reviewed in Bishop: J.M., *Cell 42*:23-38 (1985); and Weinberg, R.A., *Science 230*:770-776 (1985)). C-myc expression has been implicated as a factor in at least 10% of all human cancers.

Further, overexpression of c-myc in normal rat fibroblasts, together with expression of an activated *ras* oncogene product, transforms the fibroblasts and endows them with the ability to form tumors in living animals (Land, H. *et al., Nature 304*:596-601 (1983); Ruley, H.E., *Nature 304*:602-606 (1983)).

The function of the myc protein remains unknown despite evidence suggesting possible roles in transcriptional regulation, RNA processing, and replication. Myc is actually a family of proteins which include forms termed N-myc (DePinho, R.A. *et al., Genes Dev. 1*:1311-1326 (1987), L-myc DePinho, R.A. *et al., Genes Dev. 1*:1311-1326 (1987) and c-myc (Battet *et al., Cell 34*:779-787 (1983)).

It is clear that c-myc is involved in growth control and differentiation (Alt, F.W. *et al., Cold Spring Harbor Symp. Quant. Biol. 51*:931-941 (1986); Kelly, K. *et al., Annu. Rev. Immunol. 4*:327-328 (1986)). Recent studies suggest that oncoproteins such as c-myc alter gene expression and immortalize cells by regulating the promoter activity of specific target genes and thus activating or repressing transcription of those target genes (see, for example, Varmus, H.E., *Science 238*:1337-1339 (1987)); Kingston, R.E. *et al., Cell 41*:3-5 (1985); Bishop, J.M., *Cell 42*:23-38 (1985); Weinberg, R.A., *Science 230*:770-776 (1985)).

### II. Regulatory Proteins

Many regulatory proteins are heterodimers, that is, they are composed of two different peptide chains which interact to generate the native protein. Among such regulatory proteins are DNA binding proteins which are capable of binding to specific DNA sequences and thereby regulating transcription of DNA into RNA. The dimerization of such proteins is necessary in order for these proteins to exhibit such binding specificity.

DNA-binding sites have been shown to be interchangeable thereby leading to the recognition of an DNA region corresponding to the newly introduced DNA-binding site (Wharton et al., Nature Vol. 313, 15 August 1985, 601-605; Elledge et al., PNAS USA, Vol. 86, May 1989; 3689-3693).

Heretodimeric DNA-binding proteins have been shown to detect a hybrid nonsymmetric 434/P22-operator with high affinity (PNAS USA, Vol. 85, Aug. 1988, 5834-5839).

A large number of transcriptional regulatory proteins have been identified: Myc, Fos, Jun, Ebp, Fra-1, Jun-B, Spl, H2TF-1/NF-κB-like protein, PRDI, TDF, GLI, Evi-1, the glucocorticoid receptor, the estrogen receptor, the progesterone receptor, the thyroid hormone receptor (c-erbA) and ZIF/268, OTF-1(OCT1), OTF-2(OCT2) and PIT-1; the yeast proteins GCN4, GAL4, HAP1, ADR1, SWI5, ARGRII and LAC9, mating type factors NATα1, MATa2 and MATa1; the Neurospora proteins cys-3 and possibly cpc-1; and the Drosophila protein bsg 25D, kruppel, snail, hunchback, serendipity, and suppressor of hairy wing, antennapedia, ultrabithorax, paired, fushi tarazu, cut, and engrailed. Eukaryotic transcriptional regulatory proteins, and the methods used to characterize such proteins, have been recently reviewed (Johnson, P. F. *et al., Annu. Rev. Bioch. 58*:799-839 (1989)).

Members of the mammalian transcriptional regulatory protein families Jun/Fos and ATF/CREB only bind to DNA as dimers. The proteins in these families are "leucine zipper" proteins which contain a region rich in basic amino acids followed by a stretch of about 35 amino acids which contains 4-5 leucine residues separated form each other by 6 amino acids (the "leucine zipper" region). Collectively, the combination of a basic region and the leucine zipper region is termed the bZIP domain.

Generally, it is the basic region which has been found to be predominantly involved in contacting DNA whereas the zipper region mediates the dimerization. Many dimeric combinations are possible, however, the particular nature of the zipper specifies which partnerships are permissible (Abel, T. *et al., Nature 341*:24-25 (1989)).

Another large family of proteins contains the DNA binding/dimerization motif known as the basic helix-loop-helix motif (bHLH) (Jones, N., *Cell 61*:9-11 (1990)). A bHLH protein generally contains a basic N-terminus followed by a helix-loop-helix structure, two short amphipathic helices containing hydrophobic residues at every third or fourth position. The sequence of the basic region characteristically reveals no indication of an amphipathic helix. The intervening loop region usually contains one or more helix-breaking residues.

The bHLH motif was first detected in two proteins, E12 and E47, that bind to a specific "E box" DNA enhancer sequence found in immunoglobulin enhancers (Murre C. *et al., Cell 56*:777-783 (1989)). E motifs generally are double stranded variants of the 5'-CAGGTGGC-3' consensus sequence. For example, the µE1 motif is GTCAAGATGGC, µE2 motif is AGCAGCTGGC, µE3 is GTCATGTGGC, µE is TGCAGGTGT (Murre, C. *et al., Cell 56*:777-783 (1989)). Like many transcriptional factors, peptides containing the bHLH motif often dimerize with each other, either as a homodimer which contains two identical peptides or as a heterodimer which contains two different peptides. Examples of heterodimeric complex of two bHLH proteins binding DNA with a greater efficiency than homodimeric complexes of either peptide in the heterodimer are known (Murre C. *et al., Cell 56*:777-783 (1989); Murre, C *et al., Cell 58*:537-544 (1989)).

Myc is a bHLH protein and the bHLH domain of c-myc is encoded in c-myc amino acids 255-410. The sequence homology between the proteins expressed by the three *myc* genes (human N-myc 393-437, human c*-myc* 346-401, and human L*-myc* 289-338) and other genes which contain a bHLH domain have been compared (Murre C. *et al., Cell 56*:777-783 (1989)).

In the absence of partner proteins with which it can dimerize, a homodimer of two chains of myc does not bind to the µE2 DNA. Thus, it is desirable to identify the partners which direct myc DNA binding and compounds which inhibit myc activity by inhibiting such myc partner interaction. Proteins such as myc which contain the bHLH motif also possess the ability to dimerize with other bHLH motif proteins so it is highly likely that myc partners will also contain the bHLH motif. By inhibiting such interactions, inhibition and/or control of myc-induced cell growth may be achieved. Administration of inhibitors of myc partner formation would provide therapeutic benefits in the treatment of diseases in which expression and activity of myc is a factor in promoting cell growth or in maintaining the cell in a transformed state.

However, to date, no myc inhibitors have been identified. The identification of such inhibitors has suffered for lack of a simple, inexpensive and reliable screening assay which could rapidly identify potential inhibitors and active derivatives thereof. Thus a need still exists for rapid, economical screening assays which identify specific inhibitors of oncogene activity.

### SUMMARY OF THE INVENTION

Recognizing the potential importance of inhibitors of oncoproteins in the therapeutic treatment of many forms of cancer, and cognizant of the lack of a simple assay system in which such inhibitors might be identified, the inventors have investigated the use of chimeric oncogene constructs in *in vitro* assays in prokaryotic hosts as a model system in which to identify agents which alter oncogene expression.

These efforts have culminated in the development of a simple, inexpensive assay which can be used to identify protein partners in general, and partners of transcriptional regulatory proteins in particular.

The methods of the invention are especially useful for the identification of partners which influence transcriptional regulatory proteins, and especially oncoprotein activity.

The method of the invention further provides a method of identifying, isolating and characterizing inhibitors of such partner formation and especially inhibitors of oncoprotein activity.

The invention further provides a quick, reliable and accurate method for objectively classifying compounds, including human pharmaceuticals, as an inhibitor of oncogene activity.

The method of the invention further provides a method of identifying protein partners by their ability to disrupt λ*cI* induced repression of lytic growth in bacterial hosts which express fusion proteins containing the *cI* DNA binding domain and the partner B dimerization domain. The partners which are so identified are already in a cloned form, and easily amenable to further characterization.

### BRIEF DESCRIPTION OF THE DRAWINGS

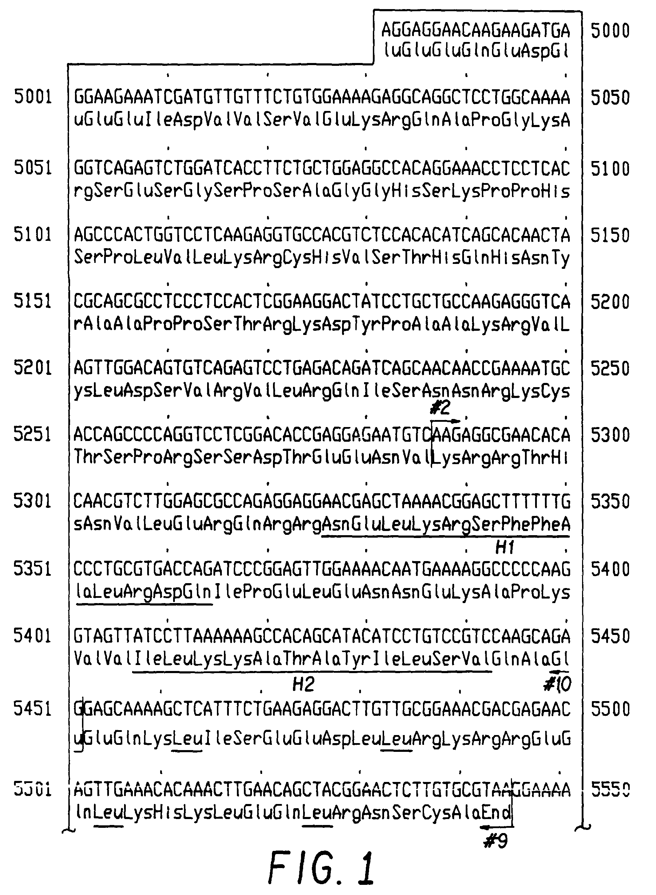

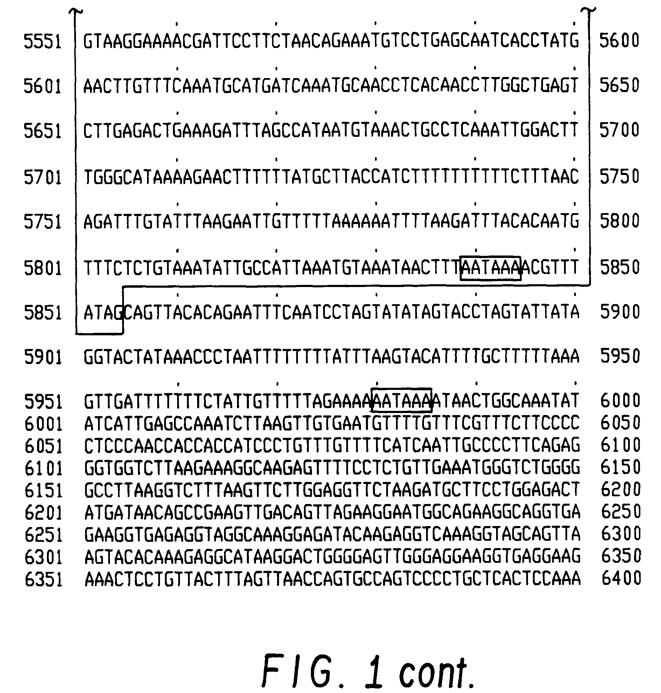

Figure 1 shows the sequence of human c-myc exon 3 and the sites used to synthesize the HLH/LZ and HLH fragments of c-myc.

### DESCRIPTION OF THE PREFERRED EMBODIMENTS

In the description that follows, a number of terms used in recombinant DNA technology are extensively utilized. In order to provide a clearer and more consistent understanding of the specification and claims, including the scope to be given such terms, the following definitions are provided.

Operably-linked. As used herein, two macromolecular elements are operably-linked when the two macromolecular elements are physically arranged such that factors which influence the activity of the first element cause the first element to induce an effect on the second element.

Fusion protein. As used herein, "fusion protein" is a hybrid protein which has been constructed to contain domains from two different proteins.

The term "fusion protein gene" is meant to refer to a DNA sequence which codes for a fusion protein, including, where appropriate, the transcriptional and translational regulatory elements thereof.

Variant. A "variant" of a fusion protein is a protein which contains an amino acid sequence that is substantially similar to, but not identical to, the amino acid sequence of a fusion protein constructed from naturally-occurring domains, that is, domains containing the native with the amino acid sequence.

By a "substantially similar" amino acid sequence is meant an amino acid sequence which is highly homologous to, but not identical to, the amino acid sequence found in a fusion protein. Highly homologous amino acid sequences include sequences of 80% or more homology, and possibly lower homology, especially if the homology is concentrated in domains of interest.

Functional Derivative. A "functional derivative" of a fusion protein is a protein which possesses an ability to dimerize with a partner protein and or, an ability to bind to a desired DNA target, that is substantially similar to the ability of the fusion protein constructs of the invention to dimerize. By "substantially similar" is meant that the above-described biological activities are qualitatively similar to the fusion proteins of the invention but quantitatively different. For example, a functional derivative of a fusion protein might recognize the same target as the fusion protein, or form heterodimers with the same partner protein, but not with the same affinity.

As used herein, for example, a peptide is said to be a "functional derivative" when it contains the amino acid backbone of the fusion protein plus additional chemical moieties not usually a part of a fusion protein. Such moieties may improve the derivative's solubility, absorption, biological half-life, etc. The moieties may alternatively decrease the toxicity of the derivative, or eliminate or attenuate any undesirable side effect of the derivative, etc. Moieties capable of mediating such effects are disclosed in Remington's Pharmaceutical Sciences (1980). Procedures for coupling such moieties to a molecule are well known in the art.

A functional derivative of a fusion protein may or may not contain post-translational modifications such as covalently linked carbohydrate, depending on the necessity of such modifications for the performance of the methods of the invention.

The term "functional derivative" is intended to encompass functional "fragments," "variants," "analogues," or "chemical derivatives" of a molecule.

Promoter. A "promoter" is a DNA sequence located proximal to the start of transcription at the 5' end of the transcribed sequence, at which RNA polymerase binds or initiates transcription. The promoter may contain multiple regulatory elements which interact in modulating transcription of the operably-linked gene.

Expression. Expression is the process by which the information encoded within a gene is transcribed and translated into protein.

A nucleic acid molecule, such as a DNA or gene is said to be "capable of expressing" a polypeptide if the molecule contains the sequences which code for the polypeptide and the expression control sequences which, in the appropriate host environment, provide the ability to transcribe, process and translate the genetic information contained in the DNA into a protein product, and if such expression control sequences are operably-linked to the nucleotide sequence which encodes the polypeptide.

Cloning vehicle. A "cloning vehicle" is any molecular entity which is capable of providing a nucleic acid sequence to a host cell for cloning purposes. Examples of cloning vehicles include plasmids or phage genomes. A plasmid which can replicate autonomously in the host cell is especially desired. Alternatively, a nucleic acid molecule which can insert into the host cell's chromosomal DNA is especially useful.

Cloning vehicles are often characterized by one or a small number of endonuclease recognition sites at which such DNA sequences may be cut in a determinable fashion without loss of an essential biological function of the vehicle, and into which DNA may be spliced in order to bring about its replication and cloning.

The cloning vehicle may further contain a marker suitable for use in the identification of cells transformed with the cloning vehicle. Markers, fcr example, are tetracycline resistance or ampicillin resistance. The word "vector" is sometimes used for "cloning vehicle."

Expression vehicle. An "expression vehicle" is a vehicle or vector similar to a cloning vehicle but is especially designed to provide sequences capable of expressing the cloned gene after transformation into a host.

In an expression vehicle, the gene to be cloned is operably-linked to certain control sequences such as promoter sequences. Expression control sequences will vary depending on whether the vector is designed to express the operably-linked gene in a prokaryotic or eukaryotic host and may additionally contain transcriptional elements host specific elements such as operator elements, upstream activator regions, enhancer elements, termination sequences, tissue-specificity elements, and/or translational initiation and termination sites.

Host. By "host" is meant any organism that is the recipient of a cloning or expression vehicle.

Response. The term "response" is intended to refer to a change in any parameter which can be used to measure and describe the effect of a compound on the activity of an protein. The response may be revealed as a physical change (such as a change in phenotype) or, it may be revealed as a molecular change (such as a change in a reaction rate or affinity constant). Detection of the response may be performed by any means appropriate.

Compound. The term "compound" is intended to refer to a chemical entity, whether in the solid, liquid, or gaseous phase. The term should be read to include synthetic compounds, natural products and macromolecular entities such as polypeptides, polynucleotides, or lipids, and also small entities such as neurotransmitters, ligands, hormones or elemental compounds.

Bioactive Compound. The term "bioactive compound" is intended to refer to any compound which induces a measurable response in the assays of the invention.

Heterodimeric proteins are proteins which contain two different polypeptide chains that associate with one another due to, for example, hydrogen bonding, ionic interactions, hydrophobic interactions, disulfide bonds, and the like, but which are not bound to one another by an amino acid linkage. The two polypeptide chains of a heterodimeric protein are herein referred to as "partners" of one another. Heterodimeric transcription regulatory proteins have been found to possess a discrete domain which is necessary for dimerization to occur. A second discrete domain is necessary for DNA binding to occur.

These observations have been explained by the inventors to permit the identification of a partner of a heterodimeric protein. Such protein partners may be identified by construction of a chimeric peptide which retains its own dimerization domain but which contains a DNA binding domain capable of conferring a phenotypic expression upon a host cell (preferably a bacterial host cell, such as E. coli). By monitoring such phenotype, formation and activity of a heterodimer may be monitored. The most preferred chimeric peptides contain a dimerization domain of a putative heterodimer protein partner and the DNA binding domain of a bacteriophage repressor, such as bacteriophage lambda (λ) repressor.

Bacteriophage λ possesses the ability to infect a bacterial host and either (a) replicate and grow in an infectious, lytic manner (the lytic cycle) or (b) integrate into the host's chromosome in a noninfectious, lysogenic manner (the lysogenic cycle). When integrated as a part of the bacterial chromosome, the inert phage DNA is referred to as a prophage. By virtue of their possession of prophage, lysogenic bacteria are immune to super infection by further phage particles of the same type. Bacteriophage lambda is a member of a family of lambdoid bacteriophages (such as 434, 21, and ø80). These are all equivalents of λ for the purposes of this invention.

When λ is grown in a lytic manner in infected bacteria, the bacteria are ultimately lysed. This lysis results in a clearing of the turbidity or opaque appearance associated with the presence of a bacterial culture. The growth and replication activity of a phage which lyses bacterial cells in its lytic cycle may be assayed by examining its ability lyse bacteria, thus forming plaques (clear areas in which the bacteria have been lysed) in a bacterial culture grown on agar *(The Bacteriophage Lambda,* Hershey, A.D., ed., Cold Spring Harbor Laboratory, New York (1971); *Lambda II,* Hendrix, R.W. *et al.,* eds., Cold Spring Harbor Laboratory, New York, (1983); (Maniatis, T. et al., Molecular Cloning (A Laboratory Manual), 2nd edition, Cold Spring Harbor Laboratory, New York (1988)).

The λ*cI* gene codes for a repressor protein, *cI,* which is necessary to maintain lysogeny. In its native state, *cI* is a homodimer (a dimer of two identical chains) and tight binding of the repressor to λ DNA requires the dimeric state. *cI* binds to two operators which control transcription of adjacent genes whose products are needed for the expression of the genes responsible for lytic development. By binding to these operators, the *cI* repressor prevents transcription of all λ genes except its own. The complete amino acid and nucleotide sequence of the cI gene is known *(Lambda II,* Hendrix, R.W. *et al.,* eds., Cold Spring Harbor Laboratory, New York, (1983)).

In the constructs of the invention, a fusion protein is created which contains the DNA binding domain of *cI* (the N-terminal 112 amino acids of λ*cI*) and the dimerization domain of a putative heterodimer protein partner. In a highly preferred embodiment, the dimerization domain, the bHLH region, of c-myc is used (amino acids 255-410 of c-myc). If desired, other dimerization domains may be used.

Dimerization domains may be predicted by analysis of the three-dimensional structure of a protein using the amino acid sequence and computer analysis techniques commonly known in the art, for example, the Chou-Fasman algorithm. Such techniques allow for the identification of helical domains and other areas of interest, for examples, hydrophobic or hydrophilic domains, in the peptide structure.

The HLH dimerization domain in a protein can be defined by comparison of a amino acid sequence with that of ten known HLH dimerization domains (amino acids 336-393 in E12, 336-393 in E47, 554-613 in *daughterless,* 357-407 in *twist,* 393-437 in human N-*myc,* 289-338 in human L-*myc*, 346-401 in human c-*myc,* 108-164 in *MyoD,* and genes of the *achaete-scute* locus: 101-167 of T4, 26-95 of T5 (Murre, C. *et al., Cell 56*:777-783 (19889)). The HLH dimerization domain contains two amphipathic helices separated by an intervening loop. The first helix contains 12 amino acids and the second helix contains 13 amino acids. Certain amino acids appear to be conserved in the HLH format, especially the hydrophobic residues which are present in the helices. Comparisons of the two sequences named above shows that there are five virtually identical hydrophilic residues within the 5' end of the homologous region and a set of mainly hydrophobic residues located in two short segments that are separated form one another by a sequence that generally contains prolines or clustered glycines.

A leucine zipper domain is usually approximately 35 amino acids long and contains a repeating heptad array of leucine residues and an exceedingly high density.of oppositely charged amino acids (acidics and basics) juxtaposed in a manner suitable for intrahelical ion pairing. It is thought that the leucines extending from the helix of one polypeptide interdigitate with those of the analogous helix of a second peptide (the partner) and form the interlock termed the leucine zipper.

The fusion protein of the invention is constructed as a recombinant DNA molecule which is capable of expressing the fusion protein in a bacterial host. Transformation of *E. coli* hosts with a recombinant λ construct capable of expressing such a fusion protein results in immunity of the bacterial host due to dimerization of the fusion protein, and especially, the *cI* domain, in a homodimer form that is able to bind the appropriate λ DNA operators. Therefore, after transformation and expression of the fusion protein, lambda lytic growth, or subsequent infection with λ, is prevented.

Preferably a low copy number plasmid is used along with a weak promoter (for example, the β-lactamase promoter and the lactose operator) for the transcription of the fusion gene so as not to overwhelm the bacterial host with a vast excess of the fusion protein. If a vast excess of fusion protein is synthesized, the ratio of the moles of fusion protein homodimer to fusion protein heterodimer (as described below) may be so high that the homodimer effectively maintains repression of phage growth and prevents detection of the heterodimer.

Any protein that possesses a binding domain which can form a heterodimer with the fusion protein will impair or prevent the formation of fusion protein homodimers. Such proteins can thus be identified by their ability to interfere with the repression of phage growth mediated by the fusion protein.

In one embodiment, the bacterial host which is expressing the above-described fusion protein, is transformed with a λ expression library which expresses cloned eukaryotic genes. For example, λ*gt*II packaging systems for the creation of expression libraries from mRNA which are useful in the methods of the invention are known in the art and may be obtained commercially (for example, through Promega Corporation, Madison, Wisconsin). Further, custom genomic expression libraries may also be obtained commercially.

Using the commercial kits, an oligo(dT)-primed cDNA library in λgtll may be generated with the use of cytoplasmic poly(A)-containing mRNA from any desired mammalian source. To induce expression of the cloned proteins contained therein, 10 mM IPTG (isopropyl-thiogalactoside) may be added.

Importantly, since all of the host cells produce the fusion protein λ repressor, lytic propagation of the infecting λ expression library will be repressed. Any infecting member of the λ expression library which does not express a protein having a dimerization domain which is capable of binding to the dimerization domain of the fusion protein will not impair the phage repression mediated by the fusion protein homodimer. Thus, no lytic growth of the λ phage will be found. If any member of the λ expression library does express a protein capable of interfering with the homodimerization of the fusion protein (as by forming a heterodimer with, for example, the bHLH domain of a fusion peptide, etc.), the repressor function will be lost and λ lytic growth will occur. Thus, identification of partners which form heterodimers can be easily made by screening for plaque forming phage.

The method of the invention is generally applicable for the identification of partners for any protein that forms partners with another protein, and especially heterodimers. An advantage of the method of the invention for the identification of protein partners is that the partner which is identified is one which has a higher affinity for the fusion protein than the homodimer affinity and thus is a protein which is highly likely to be an important regulator of the biological activity of the protein. Further, the partner which is identified is already in a cloned, expressing form which may be utilized to obtain larger quantities of the protein for its isolation, and further characterization by protein and molecular biology techniques known in the art.

The identification of protein partners in the expression library allows for the identification of compounds which inhibit the ability of such partners to form heterodimers, by screening for the ability of the compound to inhibit plaque formation.

For example, once a partner is identified by the appearance of clear or turbid plaques as described above, the identification of compounds which prevent or otherwise interfere with heterodimer formation of the protein partners can be identified by screening for the ability of such compounds to inhibit plaque formation. A compound which is found to inhibit plaque formation in this example would be a compound which (a) prevents the fusion protein from associating with the partner peptide which is also being expressed in the host and (b) does not prevent homodimer formation, that is, dimerization of the *cI* domains of the fusion protein. Such compound may or may not prevent the dimerization domains from interacting in the homodimer. (c) does not inhibit cell growth under the plaque assay conditions.

To ensure that the partner selected by the process above is specific for the regulation of the dimerization of the fusion protein and does not inhibit transcription in general, the λ which expresses such partner may be used to infect a bacterial host strain which expresses fusion proteins constructed with the dimerization domain from other proteins and the ability of the partner to induce lytic growth in such hosts examined. For example, when it is of interest to identify a compound which inhibits bHLH dimerization but not bZIP dimerization, a fusion protein is constructed which contains *cI* as above and the appropriate dimer-forming domain from a bZIP protein.

Partners, and compounds which inhibit the association of such partners, of any type of transcriptional regulation protein which associates into dimers may be identified by the bacterial methods of the invention.

Utilizing the above techniques, the inventors have also discovered specific partner proteins which associate with c-myc *in vivo* and which assist in c-myc binding to DNA. These partners strengthen the degree and duration of c-myc binding to its binding element, the DNA µE2 sequence. One of these partner proteins has a molecular weight of 46,000 daltons and binds to the *m*E2 sequence in the absence of c-myc.

The identification of a DNA binding domain in a protein may be performed by a variety of techniques known in the art and previously used to identify such domains (see Johnson, P. E. *et al., Annu. Rev. Biochem. 58*:799-839 (1989) for a review of such domains).

DNA binding proteins, and DNA binding domains in such proteins, are identified and purified by their affinity for DNA. For example, DNA binding may be revealed in filter hybridization experiments in which the protein (usually labelled to facilitate detection) is allowed to bind to DNA immobilized on a filter or, vice versa, in which the DNA binding site (usually labelled) is bound to a filter upon which the protein has been immobilized. The sequence specificity and affinity of such binding is revealed with DNA protection assays and gel retardation assays. Purification of such proteins may be performed utilizing sequence-specific DNA affinity chromatography techniques, that is, column chromatography with a resin derivatized with the DNA to which the domain binds. Proteolytic degradation of DNA binding proteins may be used to reveal the domain which retains the DNA binding ability.

The dimerization domain in a protein is recognized by its homology to known dimerization domains and can be predicted from the amino acid sequence of the protein utilizing computer-aided structural analysis as described above.

The binding domain and the dimerization domain are engineered into the fusion protein in a manner which does not destroy the function of either domain; that is, the DNA binding domain, when properly dimerized, can recognize the DNA element to which it naturally binds and the dimerization domain retains the ability to dimerize with its partners. One of skill in the art, by running control assays, will be able to establish that the fusion protein functions in the proper manner.

The fusion protein constructs of the invention may be extrachromosomally maintained as a plasmids, or inserted into the genome of a host cell.

The methods of the invention can be used to screen compounds in their pure form, at a variety of concentrations, and also in their impure form. The methods of the invention can also be used to identify the presence of such inhibitors in crude extracts, and to follow the purification of the inhibitors therefrom. The methods of the invention are also useful in the evaluation of the stability of the inhibitors identified as above, to evaluate the efficacy of various preparations.

Analogs of such compounds which are more permeable across bacterial host cell membranes may also be used. For example, dibutyryl derivatives often display an enhanced permeability.

The methods can also be used to identify partner and compounds which interfere with the partner of membrane-localized and/or with cytoplasmically-localized proteins if such proteins are capable of associating with the dimerization domain of the fusion protein.

The DNA sequence of the fusion protein and/or target gene may be chemically constructed or constructed by recombinant means known in the art. Methods of chemically synthesizing DNA are well known in the art *(Oligonucleotide Synthesis, A Practical Approach,* M.J. Gail, ed., IRL Press, Washington, D.C., 1094; *Synthesis and Applications of DNA and RNA,* S.A. Narang, ed., Academic Press, San Diego, CA, 1987). Because the genetic code is degenerate, more than one codon may be used to construct the DNA sequence encoding a particular amino acid (Watson, J.D., In: *Molecular Biology of the Gene,* 3rd edition, W.A. Benjamin, Inc., Menlo Park, CA, 1977, pp. 356-357).

To express the recombinant fusion constructs of the invention, transcriptional and translational signals recognizable by the host are necessary. A cloned fusion protein, obtained through the methods described above, and preferably in a double-stranded form, may be operably-linked to sequences controlling transcriptional expression in an expression vector, and introduced, for example by transformation, into a host cell to produce the recombinant fusion proteins, or functional derivatives thereof, for use in the methods of the invention.

Transcriptional initiation regulatory signals can be selected which allow for repression or activation of the expression of the gene encoding the fusion protein, so that expression of the fusion construct can be modulated, if desired. Of interest are regulatory signals which are temperature-sensitive so that by varying the temperature, expression can be repressed or initiated, or are subject to chemical regulation, for example, by a metabolite or a substrate added to the growth medium. Alternatively, the fusion construct may be constitutively expressed in the host cell.

It is necessary to express the proteins in a host wherein the ability of the protein to retain its biological function is not hindered. Expression of proteins in bacterial hosts is preferably achieved using prokaryotic regulatory signals.

Expression vectors typically contain discrete DNA elements such as, for example, (a) an origin of replication which allows for autonomous replication of the vector, or, elements which promote insertion of the vector into the host's chromosome in a stable manner, and (b) specific genes which are capable of providing phenotypic selection in transformed cells. Many appropriate expression vector systems are commercially available which are useful in the methods of the invention.

Once the vector or DNA sequence containing the construct(s) is prepared for expression, the DNA construct(s) is introduced into an appropriate host cell by any of a variety of suitable means, for example by transformation. After the introduction of the vector, recipient cells are grown in a selective medium, which selects for the growth of vector-containing cells. Expression of the cloned gene sequence(s) results in the production of the fusion protein.

If the fusion protein DNA encoding sequence and an operably-linked promoter is introduced into a recipient host cell as a non-replicating DNA (or RNA) molecule, which may either be a linear molecule or, more preferably, a closed covalent circular molecule which is incapable of autonomous replication, the expression of the fusion protein may occur through the transient expression of the introduced sequence.

Genetically stable transformants may be constructed with vector systems, or transformation systems, whereby the fusion protein DNA is integrated into the host chromosome. Such integration may occur *de novo* within the cell or be assisted by transformation with a vector which functionally inserts itself into the host chromosome, for example, with bacteriophage, transposons or other DNA elements which promote integration of DNA sequences in chromosomes.

Cells which have been transformed with the fusion protein DNA vectors of the invention are selected by also introducing one or more markers which allow for selection of host cells which contain the vector, for example, the marker may provide biocide resistance, e.g., resistance to antibiotics, or the like.

The transformed host cell can be fermented according to means known in the art to achieve optimal cell growth, and also to achieve optimal expression of the cloned fusion protein sequence fragments. Optimal expression of the fusion protein is expression which provides no more than the same moles of fusion protein subunit as the moles of the partner protein which are being expressed. However, variations in this amount are acceptable if they do not interfere with the ability of the partner, when in heterodimer form, to override the *cI* repressor activity.

It may be desired to further characterize the partner proteins of c-myc which are identified by the methods of the invention in a eukaryotic expression system. Such characterization may be performed according to the methods described in the inventor's copending U.S. patent application entitled "C-MYC SCREENING ASSAYS," Serial No. 07/210,253 , filed the same day as this application, April 19, 1990 and incorporated herein by reference.

The following examples further describe the materials and methods used in carrying out the invention. The examples are not intended to limit the invention in any manner.

### EXAMPLES

### Example 1

### Construction of c-myc Fusion Proteins

The promoter/operator regions in all of these constructs is the same and consists of the β-lactamase promoter, and the lac operator and Shine-Delgarno (S.D.) sequence. The sequence is as follows: 
GGA TCC TCT AAA TAC ATT CAA ATA AGT ATC CGC TCA TGA
BamHI -35
GAC AAT AAC GGT AAC CAG AAT TGT GAG CGC TCA CAA TTT TG
-10 BstEII
ATC GAT AGG AAA CTC GAG ATG...
ClaI S.D. XhoI +1 cI
 *cI* in each of these constructs consists of the first 330 bp (1121 amino acids) which corresponds to the N-terminal polypeptide generated by recA cleavage. It was synthesized with polymerase chain reaction (PCR) primers with *Xho*I and *Xba*I sites on the 5' and 3' ends, respectively. The promoter/operator and *cI* DNAs were cloned into pUC18 digested with *BamH*I and *Xba*I*.*

The sequence around the *Xba*I site is as follows:
5' CAG GCA GGG TCT AGA...
Gln Ala Gly *Xba*I
*cI* coding seq.

The HLH/LZ (helix-loop-helix/leucine zipper) and HLH fragments of c-myc were generated by PCR using a human c-myc cDNA as a template. HLH/LZ is a 257 bp fragment synthesized with primers starting at sites #2 and #9 (Figure 1) with XbaI and *Sal*I sites 5' and 3', respectively. HLH is a 178 bp fragment with *Xba*I and *Pst*I sites on the 5' and 3' ends, respectively; the boundaries of HLH are at sites marked #2 and #10. The primer used at site #10 included a termination codon, as does that used at site #9. Insertion of the c-myc sequences in to pU33cI was at the restriction sties corresponding to those on the indicated PCR primers.

Each of the constructs in pUC18 was subcloned into pYC177 as follows. pYC177 was digested with *Bgl*I (filled in with Klenow) and *BamH*I and each of the pUC18 based constructs was digested with *Hind*III (filled in with Klenow) and *BamH*I*.* The resulting pYC-constructs provide kanamycin resistance.

### Example 2

### Screening a cDNA expression library for protein partners able to interact with the helix-loop-helix domain of c-Myc.

To screen for proteins able to interact with the helix-loop-helix (HLH) of human c-Myc, DNA encoding amino acids 255-410 of c-Myc, which contain the basic region and HLH of the c-Myc peptide was ligated, as above, in frame, to a DNA fragment encoding the N-terminal 112 amino acids of the lambda repressor (cI) protein. The expression of this chimeric protein was placed under the control of the very weak β-lactamase promoter and the lactose operator. This expression unit was subcloned into pACYC177, a low copy number plasmid (10-15 copies/cell) with a kanamycin^{R} gene. Cells transformed with this construct were shown to be resistant to lambda phage infection by a dot plaque assay. The above construct, pYC192cIHLH, made cells resistant to phage infection by >10⁸ pfu, whereas cells expressing the N-terminal region of cI alone were infected at <10² pfu.

*E. coli* strain Y1090 transformed with pYC192cIHLH was used to screen a human tonsil/B cell λgtll expression library, constructed according to the manufacturer's recommendations (Promega). 5 x 10⁶ pfu were screened in duplicate on the above transformed strain, as well as a Y1090::λ lysogen strain. On each of test plates 800 plaques formed. On the control plates there were no plaques were plaque-purified once and a single plaque was picked and suspended in suspension medium (SM). The 160 purified plaques were grouped according to plaque size: 20 small, 70 medium, 70 large. (Four of the "small" group did not form plaques in the plaque purification step.) Each of these was then screened by a dot plaque assay on an *E. coli.* strain, JM109, transformed with the plasmid pJH370, which expresses a chimeric protein consisting of the N-terminus of cI fused to the leucine zipper domain of GCN4. Each phage was also retested on the above mentioned strain used in the primary screen, as well as the λ lysogenic strain and the parental Y1090 strain.

Phage which formed plaques on the parental Y1090 strain and the cI-Myc-expressing strain, but not on either the lysogenic strain or the cI-GCN4-expressing strain were defined as "positive". "Positives" were screened a total of twice on these four strains. In the dot plaque assay 5-20µl of the "positive" phage typically yielded 50-100 plaques. If a single, tiny plaque could be seen on cI-GCN4, then the phage was defined as "negative". By this assay, a total of 10 "positives" of the 156 phage tested were found. Of these, 3 were of the "small" group and 7 were of the "medium" group. These positives represent proteins which associate with c-myc in a manner sufficient to disrupt homodimer formation.

### Example 3

### Identification of a compound which prevent c-Myc partner heterodimerization

*E. coli* host cells which express a protein identified as a "medium" c-myc partner protein as described in Example 2 are further exposed to compounds W, X, Y, and Z and the effect of such compounds on the ability of the *l* to grow in a lytic manner is determined by looking for the ability of the compound to inhibit plaque formation in solid agar plates.

Typical results from such an experiment are shown in Table 1.

**Table 1:**

| Identification of C-myc-protein Partners | | |
|---|---|---|
| Compound | *gt*ll Partner | Plaque Formation |
| none | - | no |
| | + | yes |
| | | |
| W | - | no |
| | + | yes |
| | | |
| X | - | yes |
| | + | yes |
| | | |
| Y | - | no |
| | + | no |

The results of the above table indicate that, in the absence of the partner protein, compound W had no effect on the ability of the λ*cI* protein to form dimers and repress lytic growth. Further compound W had no effect on the ability of the partner to form heterodimers with the myc fusion protein. Therefore, compound W will not be a compound of interest.

Compound X interfered with homodimer formation and not with and heterodimer association. Therefore, compound X is not an inhibitor of c-myc function.

Compound Y is an inhibitor of heterodimer formation. Compound Y did not interfere with homodimer formation but did interfere with heterodimer formation. Therefore, compound Y is a compound which may disrupt c-myc action *in vivo.*

All references cited herein are fully incorporated by reference. Having now fully described the invention, it will be understood by those with skill in the art that the scope may be performed within a wide and equivalent range of conditions, parameters and the like, without affecting the spirit or scope of the invention or any embodiment thereof.

## Claims

1. A method for identifying and classifying a protein partner wherein said method comprises:
(a) transformation of a bacterial host cell with a genetic construct capable of expressing a fusion protein, wherein said fusion protein contains a DNA binding domain and a dimerization domain, and wherein said fusion protein forms a homodimer which represses growth of a lytic bacteriophage;
(b) transformation of said host cell of part (a) with a genetic construct capable of expressing said protein partner;
(c) culturing said host cell of part (b) under conditions which express said fusion protein and said protein partner;
(d) determining the ability of said lytic bacteriophage to induce lysis of said host cell; and
(e) classifying said protein partner on the basis of the presence or absence of said lysis.

2. A method of identifying and classifying a compound as an inhibitor of dimerization of a protein partner, wherein said method comprises:
(a) transformation of a bacterial host cell with a genetic construct capable of expressing a fusion protein, wherein said fusion protein contains a DNA binding domain and a dimerization domain, and wherein said fusion protein forms a homodimer which represses growth of a lytic bacteriophage;
(b) transformation of said host cell of part (a) with a genetic construct capable of expressing said protein partner;
(c) culturing said host cell of part (b) in the presence of said compound and under conditions which express said fusion protein and said protein partner;
(d) determining the ability of said compound to prevent protein-partner-induced growth of said lytic bacteriophage and lysis of said host cell; and
(e) classifying said compound as an inhibitor of protein partner formation on the basis of the presence or absence of said lysis.

3. The method of any one of claims 1 or 2, wherein said DNA binding domain is the DNA binding domain of the bacteriophage λ *cI* repressor protein.

4. The method of claim 4, wherein said DNA binding domain of said *cI* repressor protein is the N-terminal 112 amino acids of said repressor protein.

5. The method of any one of claims 1 or 2, wherein said phage is λ.

6. The method of any one of claims 1 or 2, wherein said dimerization domain is a bHLH domain.

7. The method of claim 6, wherein said bHLH domain is from Myc.

8. The method of claim 7, wherein said myc is c-Myc.

9. The method of claim 8, wherein said bHLH domain is amino acids 255-410 of c-Myc.

10. The method of any one of claims 1 or 2, wherein said dimerization domain is a bZIP domain.

11. The method of any one of claims 1 or 2, wherein said dimerization domain is a zinc finger domain.

## Patentansprüche

1. Verfahren zum Identifizieren und Klassifizieren eines Proteinpartners, welches Verfahren umfaßt:
(a) Transformation einer bakteriellen Wirtszelle mit einem Genkonstrukt, das zur Expression eines Fusionsproteins befähigt ist, worin das Fusionsprotein eine DNA-Bindungsdomäne und eine Dimerisationsdomäne enthält und worin das Fusionsprotein ein Homodimer ausbildet, das ein Wachstum eines lytischen Bakteriophagen unterdrückt;
(b) Transformation dieser Wirtszelle aus Teil (a) mit einem Genkonstrukt, das zur Expression des Proteinpartners befähigt ist;
(c) Vermehren dieser Wirtszelle aus (b) unter Bedingungen, welche das Fusionsprotein und den Proteinpartner exprimieren;
(d) Bestimmen des Vermögens des lytischen Bakteriophagen zum Induzieren einer Lysis der Wirtszelle; und
(e) Klassifizieren des Proteinpartners auf der Basis des Vorliegens oder Ausbleibens der Lysis.

2. Verfahren zum Identifizieren und Klassifizieren einer Verbindung als ein Inhibitor der Dimerisation eines Proteinpartners, welches Verfahren umfaßt:
(a) Transformation einer bakteriellen Wirtszelle mit einem Genkonstrukt, das zur Expression eines Fusionsproteins befähigt ist, worin das Fusionsprotein eine DNA-Bindungsdomäne und eine Dimerisationsdomäne enthält und worin das Fusionsprotein ein Homodimer ausbildet, das ein Wachstum eines lytischen Bakteriophagen unterdrückt;
(b) Transformation dieser Wirtszelle aus Teil (a) mit einem Genkonstrukt, das zur Expression des Proteinpartners befähigt ist;
(c) Vermehren dieser Wirtszelle aus (b) in Anwesenheit der genannten Verbindung und unter Bedingungen, welche das Fusionsprotein und den Proteinpartner exprimieren;
(d) Bestimmen des Vermögens der Verbindung zur Vermeidung eines durch den Proteinpartner induzierten Wachstums des lytischen Bakteriophagen und der Lysis der Wirtszelle; und
(e) Klassifizieren der Verbindung als ein Inhibitor der Proteinpartnerbildung auf der Basis des Vorliegens oder Ausbleibens der Lysis.

3. Verfahren nach einem der Ansprüche 1 oder 2, worin die DNA-Bindungsdomäne die DNA-Bindungsdomäne des Bakteriophagen λ cI-Repressorproteins ist.

4. Verfahren nach Anspruch 3, worin die DNA-Bindungsdomäne des cI-Repressorproteins die N-terminalen 112 Aminosäuren des Repressorproteins sind.

5. Verfahren nach einem der Ansprüche 1 oder 2, worin der Phage λ ist.

6. Verfahren nach einem der Ansprüche 1 oder 2, worin die Dimerisationsdomäne eine bHLH-Domäne ist.

7. Verfahren nach Anspruch 6, worin die bHLH-Domäne von Myc stammt.

8. Verfahren nach Anspruch 7, worin das Myc c-Myc ist.

9. Verfahren nach Anspruch 8, worin die bHLH-Domäne die Aminosäuren 255-410 von c-Myc sind.

10. Verfahren nach einem der Ansprüche 1 oder 2, worin die Dimerisationsdomäne eine bZIP-Domäne.

11. Verfahren nach einem der Ansprüche 1 oder 2, worin die Dimerisationsdomäne eine Zink-Fingerdomäne ist.

## Revendications

1. Procédé pour l'identification et la classification d'un partenaire de protéine, ledit procédé comprenant:
(a) la transformation d'une cellule hôte bactérienne par un produit de construction génétique capable d'exprimer une protéine de fusion, ladite protéine de fusion contenant un domaine de liaison à l'ADN et un domaine de dimérisation, et ladite protéine de fusion formant un homodimère qui réprime la croissance d'un bactériophage lytique;
(b) la transformation de ladite cellule hôte de (a) par une construction génétique capable d'exprimer ledit partenaire de protéine;
(c) la culture de ladite cellule hôte de (b), dans des conditions permettant l'expression de ladite protéine de fusion et dudit partenaire de protéine;
(d) la détermination de l'aptitude dudit bactériophate lytique à induire la lyse de ladite cellule hôte; et
(e) la classification dudit partenaire de protéine sur la base de la présence ou de l'absence de ladite lyse.

2. Procédé d'identification et de classification d'un composé en tant qu'inhibiteur de dimérisation d'un partenaire de protéine, ledit procédé comprenant:
(a) la transformation d'une cellule hôte bactérienne par un produit de construction génétique capable d'exprimer une protéine de fusion, ladite protéine de fusion contenant un domaine de liaison à l'ADN et un domaine de dimérisation, et ladite protéine de fusion formant un homodimère qui réprime la croissance d'un bactériophage lytique;
(b) la transformation de ladite cellule hôte de (a) par une construction génétique capable d'exprimer ledit partenaire de protéine;
(c) la culture de ladite cellule hôte de (b) en présence dudit composé et dans des conditions permettant l'expression de ladite protéine de fusion et dudit partenaire de protéine;
(d) la détermination de l'aptitude dudit composé à empêcher la crois.sance, induite par le partenaire de protéine, dudit bactériophage lytique et la lyse de ladite cellule hôte; et
(e) la classification dudit composé en tant qu'inhibiteur de la formation de partenaire de protéine, sur la base de la présence ou de l'absence de ladite lyse.

3. Procédé selon la revendication 1 ou 2, dans lequel ledit domaine de liaison à l'ADN est le domaine de liaison à l'ADN du répresseur cI de bactériophage λ.

4. Procédé selon la revendication 3, dans lequel ledit domaine de liaison à l'ADN dudit répresseur cI consiste en les 112 aminoacides N-terminaux dudit répresseur.

5. Procédé selon la revendication 1 ou 2, dans lequel ledit phage est le phage λ.

6. Procédé selon la revendication 1 ou 2, dans lequel ledit domaine de dimérisation est un domaine bHLH.

7. Procédé selon la revendication 6, dans lequel ledit domaine bHLH provient de Myc.

8. Procédé selon la revendication 7, dans lequel ladite Myc est c-Myc.

9. Procédé selon la revendication 8, dans lequel ledit domaine bHLH consiste en les aminoacides 255-410 de c-Myc.

10. Procédé selon la revendication 1 ou 2, dans lequel ledit domaine de dimérisation est un domaine bZIP.

11. Procédé selon la revendication 1 ou 2, dans lequel ledit domaine de dimérisation est un domaine de doigts à zinc.
